(19) Europäisches Patentamt / European Patent Office / Office européen des brevets

(11) **EP 2 656 775 B1**

(12) **EUROPEAN PATENT SPECIFICATION**

(45) Date of publication and mention
of the grant of the patent:
**20.07.2016 Bulletin 2016/29**

(51) Int Cl.:
*A61B 1/00* *(2006.01)*   *A61B 5/07* *(2006.01)*
*A61B 1/04* *(2006.01)*   *A61B 5/06* *(2006.01)*

(21) Application number: **12752226.6**

(22) Date of filing: **07.02.2012**

(86) International application number:
**PCT/JP2012/052758**

(87) International publication number:
**WO 2012/117815 (07.09.2012 Gazette 2012/36)**

(54) **POSITION DETECTING APPARATUS OF CAPSULE ENDOSCOPE AND CAPSULE ENDOSCOPE SYSTEM**

VORRICHTUNG ZUR ERKENNUNG DER POSITION EINES KAPSELFÖRMIGEN ENDOSKOPS, KAPSELFÖRMIGES ENDOSKOPSYSTEM UND PROGRAMM ZUR BESTIMMUNG DER POSITION EINES KAPSELFÖRMIGEN ENDOSKOPS

DISPOSITIF POUR LA DÉTECTION DE LA POSITION D'UN ENDOSCOPE EN FORME DE CAPSULE, SYSTÈME D'ENDOSCOPE EN FORME DE CAPSULE ET PROGRAMME DE DÉTERMINATION DE LA POSITION D'UN ENDOSCOPE EN FORME DE CAPSULE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.03.2011 JP 2011045684**

(43) Date of publication of application:
**30.10.2013 Bulletin 2013/44**

(73) Proprietor: **Olympus Corporation**
**Shibuya-ku**
**Tokyo (JP)**

(72) Inventor: **HASEGAWA, Jun**
**Tokyo 151-0072 (JP)**

(74) Representative: **Gunzelmann, Rainer**
**Wuesthoff & Wuesthoff**
**Patentanwälte PartG mbB**
**Schweigerstraße 2**
**81541 München (DE)**

(56) References cited:
**EP-A1- 1 884 184      EP-A1- 2 008 572**
**EP-A1- 2 082 680      JP-A- 2007 195 586**
**JP-A- 2007 283 001      US-A1- 2008 242 931**

**Description**

Field

The present invention relates to a position detecting apparatus and a capsule endoscope system for receiving a wireless signal transmitted from a capsule endoscope within a subject by a receiving device that is disposed outside the subject and detecting a position of the capsule endoscope based on the received wireless signal.

Background

Conventionally, in the field of endoscopes, known are capsule endoscopes in which an imaging function, a radio communication function, and the like are embedded in a capsule-shaped casing formed in a size that is able to introduced into a gastrointestinal tract of a subject such as a patient. After being swallowed from the subject's mouth, the capsule endoscope moves within the subject such as the gastrointestinal tract by peristalsis motion and the like. Then, it sequentially captures the inside of the subject to generate image data and sequentially transmits the image data in a wireless manner.

The image data wirelessly transmitted from the capsule endoscope in such a way is received at the receiving device provided outside the subject. The image data received by the receiving device is stored in a memory embedded in the receiving device. Upon completion of the examination, the image data accumulated in the memory of the receiving device is inputted to the image display device. The observer such as a doctor or a nurse observes the internal organ displayed on the image display device and the subject is diagnosed.

Because the capsule endoscope moves by the peristalsis motion in the body cavity, it is necessary to correctly recognize at which position in the body cavity the image data transmitted by the capsule endoscope is taken.

In this regard, a capsule endoscope is disclosed that receives the electromagnetic wave transmitted by the capsule endoscope by a plurality of receiving antennas outside the body cavity and estimates the position and orientation of the capsule endoscope by using a Gaussian Newton method based on the received strength of a plurality of received wireless signals (See Patent Literature 1, for example).

Further, a capsule endoscope is disclosed that provides a sensor for collecting intra-subject information and recognizes the position and the like of the capsule endoscope that is inside the subject based on the information collected by the sensor (See Patent Literature 2, for example).

Citation List

Patent Literature

    Patent Literature 1: Japanese Laid-open Patent Publication No. 2007-000608
    Patent Literature 2: Japanese National Publication of International Patent Application No. 2010-524557

EP 2 008 572 A1 discloses a technique for estimating the position of an antenna incorporated in a capsule-type endoscope that moves in a body using a plurality of antennae. The distance between two positions estimated at adjacent times falls within a predetermined value, pieces of information for these positions are related to each other and stored in a memory as connection information. Subsequently, processing for searching for a route from the connection information stored in the memory and calculating a track is performed.

EP 1 884 184 A1 discloses a capsule medical system for detecting the location at which biological information, such as an image captured in a living body, is acquired. A circular loop antenna incorporated in an endoscopic capsule traveling in a living body transmits a high-frequency signal and a plurality of antennas disposed on a body surface of the living body receives the signal. A CPU defines the initial location and orientation of the antenna. The CPU then performs an estimation process on the initial location and orientation so as to compute new estimated location and orientation and update the initial location and orientation to the new location and orientation. The CPU similarly performs the estimation process on the updated location and orientation. The CPU performs accurate location estimation by repeatedly performing the estimation process until the amount of shift of the location computed and updated by the estimation process reaches a sufficiently small value.

EP 2 082 680 A1 discloses a swallowable capsule endoscope. It includes an image pickup device for picking up an image in a body. A transmitter externally transmits information of the image. By use of an acceleration sensor, a capsule moving speed is detected in the body. A CPU changes a pixel number of the image according to information on the capsule moving speed. The CPU sets the pixel number lower according to a low capsule moving speed. Also, the CPU sets the pixel number lower according to a low capsule moving distance, and/or according to a high similarity degree between plural consecutive images.

US 2008/0242931 A1 discloses a receiver of capsule endoscopic system that downloads diagnostic information of a patient obtained by a past diagnosis from a data storage of work station. A data analyzer of the receiver compares the diagnostic information and present information obtained by use of a capsule endoscope during endoscopy. A CPU produces a control command in which a frame rate of image shooting by use of the capsule endoscope is set based on an analysis result of the data analyzer. The produced control command is wirelessly transmitted via a radio wave from the receiver. The capsule endoscope wirelessly receives the control command from the receiver via the radio wave, and performs image shooting with the frame rate set by the control command.

Summary

Technical Problem

However, if the position and orientation of the capsule endoscope is estimated by using Gaussian Newton method disclosed in Patent Literature 1, steepest descent method, or Marquardt algorithm, the position and orientation is estimated while being updated, which makes it difficult to achieve high-speed processing because of a great amount of calculation.

If the sensor is provided inside the capsule endoscope as disclosed in Patent Literature 2, the device configuration within the capsule endoscope is complicated, which makes it difficult to achieve a small-sized capsule endoscope. Furthermore, power supply to the sensor causes an increase in power consumption.

The present invention has been made in view of the foregoing and it is an object of the present invention to achieve a small-sized capsule endoscope, and to provide a receiving device and a capsule endoscope system that are capable of performing, at high speed, a process for estimating a position and orientation of the capsule endoscope in a body cavity.

Solution to Problem

In order to solve the above problems and achieve the object, a position detecting apparatus of a capsule endoscope according to the present invention, which is defined in independent claim 1 and the dependent claims, includes: a receiving antenna unit for receiving, by a plurality of receiving antennas, a wireless signal transmitted from a capsule endoscope within a subject; a storage unit for storing a theoretical electric field strength of the wireless signal received by each of the receiving antennas, the theoretical electric field strength depending on a position or a position and orientation of the capsule endoscope in the subject; an electric field strength comparing unit for comparing a specified value calculated by using a difference between a received electric field strength of the wireless signal received by each of the receiving antennas and the theoretical electric field strength stored in the storage unit; and a position determination unit for determining a position and orientation of the capsule endoscope where image data has been taken, based on a comparison result by the comparing unit.

Therein, the storage unit is configured to store a theoretical electric field strength according to an orientation of the capsule endoscope for each of a plurality of subregions into which a region within the subject where the capsule endoscope can be present is divided.

The electric field strength comparing unit is configured to calculate, as a first estimation step, a residual sum of squares between the theoretical electric field strength stored in the storage unit and the received electric field strength for each center position of the subregions and for each orientation, and the position determination unit is configured to determine a position and orientation of the capsule endoscope in the first estimation step, based on a comparison result by the electric field strength comparing unit. Therein, the determined position is a center position of a subregion and the determined position and orientation have the smallest one of the residual sum of squares calculated by the electric field strength comparing unit.

The electric field strength comparing unit is configured to calculate, as a second estimation step, a residual sum of squares between the theoretical electric field strength stored in the storage unit and the received electric field strength for a limited region including the position of the capsule endoscope determined in the first step and for each orientation of the capsule endoscope. The position determination unit is configured to determine as a final position and orientation of the capsule endoscope in the second estimation step, the position and orientation which has the smallest sum of residual squares calculated by the electric field strength comparing unit.

In an embodiment of the position detecting apparatus of the capsule endoscope according to the present invention, the comparing unit extracts, for each image data, a specified number of regions and orientations in ascending order of the residual sums of squares, as a candidate of the position and orientation of the capsule endoscope, and based on a distance between candidate positions and/or the residual sum of squares of the image data for a previous timing and a subsequent timing, the determination unit determines the position or the position and orientation of the capsule endoscope where each image data has been taken.

In an embodiment of the position detecting apparatus of the capsule endoscope according to the present invention, the receiving antenna unit has a sheet shape on which the plurality of receiving antennas are provided.

According to an embodiment, the position detecting apparatus of the capsule endoscope according to the present invention further includes a trajectory calculation unit for calculating a trajectory of the capsule endoscope based on the position of the capsule endoscope determined by the determination unit.

A capsule endoscope system according to the present invention includes: a capsule endoscope for obtaining image data of an inside of a subject; the above-described position detecting apparatus for receiving the image data transmitted from the capsule endoscope and estimating a position and orientation of the capsule endoscope where the received image data has been taken; and an image display unit for obtaining the image data and position information of the image data from the receiving antenna and the position detecting apparatus and for displaying the obtained image data position information.

In the capsule endoscope system according to the present invention, the image display unit displays the image data and a movement trajectory in the subject of the capsule endoscope calculated by the trajectory calculation unit.

Further, a position detecting program of a capsule endoscope is disclosed that causes a position detecting apparatus for receiving image data transmitted from the capsule endoscope in a subject and estimating a position and orientation of the capsule endoscope where the received image data has been taken, to execute: a wireless signal obtaining step of obtaining a wireless signal transmitted from the capsule endoscope, the wireless signal being received by a plurality of receiving antennas of a receiving antenna unit; a theoretical strength obtaining step of obtaining, from a storage unit, a theoretical electric field strength of the wireless signal received by each of the receiving antennas, the theoretical electric field strength depending on a position or a position and orientation of the capsule endoscope in the subject; a comparing step of comparing a specified value calculated by using a difference between a received electric field strength of the wireless signal received by each of the receiving antennas and the theoretical electric field strength stored in the storage unit; and a position determination step of determining a position or a position and orientation of the capsule endoscope where image data has been taken, based on a comparison result in the comparing step.

Advantageous Effects of Invention

According to the present invention, a theoretical electric field strength which is estimated to be received by each receiving antenna, at a possible position of a capsule endoscope in a subject, is stored in advance, and a position and orientation of the capsule endoscope is determined based on a difference between the stored theoretical electric field strength and an electric field strength of a wireless signal which is actually received by each receiving antenna. Therefore, it is possible to perform a position estimation process at high speed.

Brief Description of Drawings

FIG. 1 is a schematic diagram illustrating a schematic configuration of a capsule endoscope system using a receiving device according to a first example;
FIG. 2 is a cross-sectional view illustrating a schematic internal configuration of the capsule endoscope;
FIG. 3 is a block diagram illustrating a schematic configuration of the receiving device according to the first example;
FIG. 4A is a schematic diagram for explaining position detection of the capsule endoscope;
FIG. 4B is a schematic diagram in which a region of FIG. 4A is divided into four regions in each of x, y, and z directions;
FIG. 5 is a schematic view illustrating electromagnetic field components in a arbitrary position with respect to an antenna (using a circle coil) of the capsule endoscope;
FIG. 6 is a schematic view illustrating that the electromagnetic field attenuates when propagating in a medium;
FIG. 7 is a schematic view illustrating a relationship between an electric field generated by the capsule endoscope and a direction of one of receiving antennas of a receiving antenna unit;
FIG. 8A is a schematic diagram in which the region where the capsule endoscope is present is divided into three regions in each of the x, y, and z directions;
FIG. 8B is a schematic diagram in which one of the regions of FIG. 8A is further divided into three regions in each of the x, y, and z directions;
FIG. 9 is a block diagram illustrating a schematic configuration of a receiving device according to a second embodiment of the present invention;
FIG. 10 is a flowchart illustrating an outline of a trajectory calculation process performed by a trajectory calculation unit;
FIG. 11 is a schematic view illustrating a plurality of candidate positions that have been position-estimated for a plurality of image data taken at a previous timing and a subsequent timing;

FIG. 12 is a flowchart of a trajectory estimation process;

FIG. 13A is a display example on an image display device that shows a trajectory of the capsule endoscope within a subject calculated by the receiving device of the second embodiment; and

FIG. 13B is a display example on the image display device that shows a trajectory of the capsule endoscope within the subject calculated by the receiving device of the second embodiment.

Description of an Example and Embodiments

A position detecting apparatus and a capsule endoscope system according to the first example will be described below by referring to the

drawings. It is noted that, in the following description, while a capsule endoscope system including a capsule endoscope that is introduced in a subject's body and captures in-vivo images of the subject will be exemplified as a first example of a position detecting apparatus and a capsule endoscope system, the present invention is not limited to the example.

(First Example)

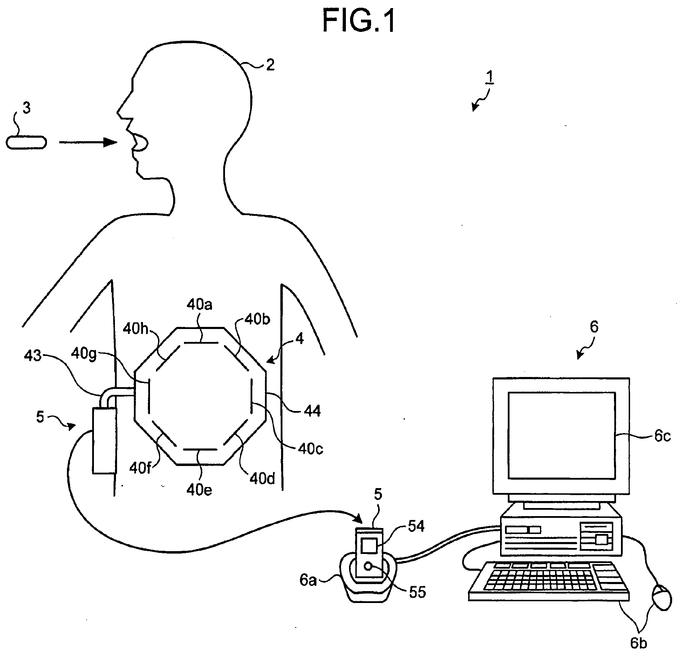

FIG. 1 is a schematic diagram illustrating a schematic configuration of a capsule endoscope system 1 with a position detecting apparatus according to a first example. As illustrated in FIG. 1, the capsule endoscope system 1 includes a capsule endoscope 3 that captures in-vivo images of a subject 2, a receiving device 5 that receives a wireless signal transmitted by the capsule endoscope 3 introduced into the subject 2 and receives a capturing position of the image data of the inside of the subject 2 captured by the capsule endoscope 3, and an image display device 6 that displays an image corresponding to the image data of the inside of the subject 2 captured by the capsule endoscope 3.

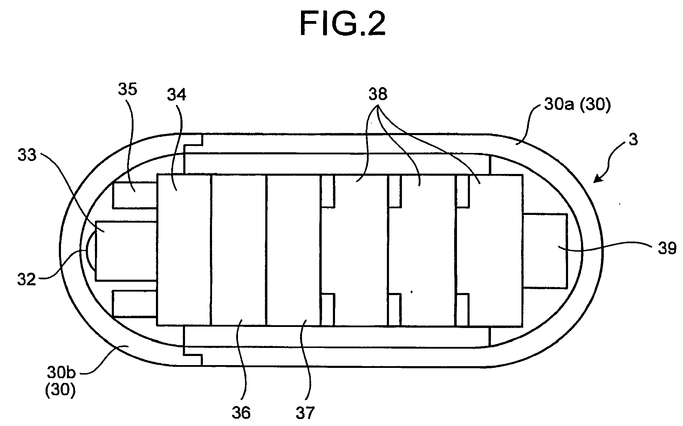

FIG. 2 is a cross-sectional view illustrating the schematic internal configuration of the capsule endoscope 3. As illustrated in FIG. 2, the capsule endoscope 3 is contained in a capsule container 30 (casing) having an approximately cylindrical or semi-ellipse sphere container 30a, one end of which is semi-sphere dome shape and the other end of which is opened, and an semi-sphere optical dome 30b that is fitted into the opening of the container 30a to seal the container 30a in a watertight manner. The capsule container 30 (30a, 30b) is, for example, of a size that the subject 2 can swallow. Further, in the first example, at least the optical dome 30b is formed with a transparent material.

Further, the capsule endoscope 3 includes an objective lens 32 for forming an image of the light entered through the optical dome 30b, a lens frame 33 by which the objective lens 32 is attached, an imaging unit 34 for converting an optical signal entered from the objective lens 32 into an electrical signal to form a capturing signal, a lighting unit 35 for lighting the inside of the subject 2 at the imaging, a circuit board 36 on which a processing circuit and the like for driving the imaging unit 34 and the lighting unit 35, respectively, and generating an image signal from the imaging signal entered from the imaging unit 34 are formed, a transceiving circuit 37 for transmitting the image signal and receiving a signal from the receiving device 5 and the like that are disposed outside the body cavity, and a plurality of button batteries 38 for supplying power to respective function units described above.

The capsule endoscope 3 passes through the esophagus in the subject 2 after swallowed into the subject and moves inside the body cavity by the peristalsis motion of the gastrointestinal tract cavity. The capsule endoscope 3 sequentially captures the inside of the body cavity of the subject 2 at a short interval of time such as an interval of 0.5 second, while moving inside the body cavity, and generates the image data of the inside of the captured subject 2 to sequentially transmit it to the receiving device 5. In the first example, although it is possible to perform the position estimation process by the image signal of the image data taken by the imaging unit 34 of the capsule endoscope 3, it is preferable to generate a transmission signal including the captured image signal and the received strength detecting signal for the position detection of the capsule endoscope 3 and perform the position detection process by using the received strength detection signal whose received strength can be easily detected.

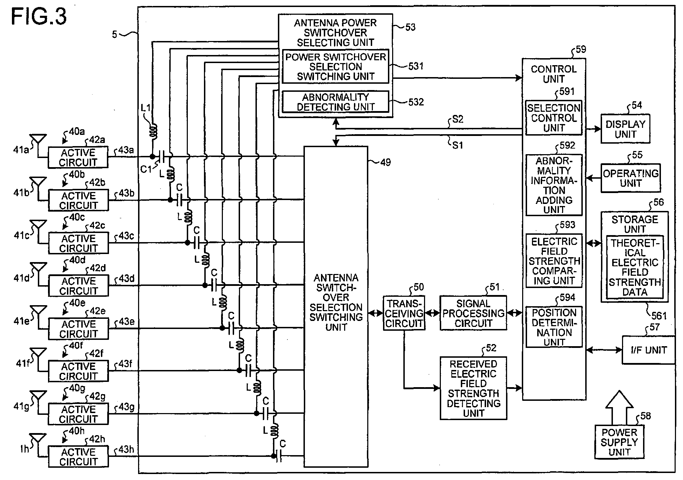

The position detecting apparatus includes a sheet-shaped receiving antenna unit 4 on which a plurality of receiving antennas 40 (40a, 40b, 40c, 40d, 40e, 40f, 40g, 40h) are provided and the receiving device 5. The receiving device 5 is connected to the receiving antenna unit 4 by an antenna cable 43. The receiving device 5 receives the wireless signal transmitted from the capsule endoscope 3 through each of the receiving antennas 40a to 40h. The receiving device 5 detects the received electric field strength of the wireless signal received from the capsule endoscope 3 for each of the receiving antennas 40a to 40h and obtains the image data inside the subject 2 based on the received wireless signal. The receiving device 5 associates the received electric field strength information, the time information indicating the time, and so on with the received image data for each of the receiving antennas 40a to 40h and stores them in a storage unit (see FIG. 3) described later.

While the capturing is being performed by the capsule endoscope 3, the receiving device 5 is carried by the subject 2, for example, after it is introduced into the subject 2 from its mouse and passes through the gastrointestinal tract before excreted from the subject 2. Upon the completion of the examination by the capsule endoscope 3, the receiving device 5 is removed from the subject 2 and connected to the image display device 6 for transferring the information such as the image data received from the capsule endoscope 3.

The receiving antennas 40a to 40h are disposed at specified positions on a sheet 44. For example, the specified positions correspond to organs of the subject 2 along a path of the endoscope 3 when the receiving antenna unit 4 is attached to the subject 2. It is noted that the arrangement of the receiving antennas 40a to 40h may be changed according to the purpose such as the examination, the diagnosis, and the like. Although eight receiving antennas are used in the present example, it is not necessary to limit the number of the receiving antennas to eight, and the number may be less than eight or greater than eight.

The image display device 6 is configured with a workstation or a personal computer having a monitor unit 6c such as a liquid crystal display. The image display device 6 displays the image corresponding to the image data of the inside of the subject 2 obtained through the receiving device 5. A cradle 6a and an operation input device 6b such as a keyboard, a mouse, and so on are connected to the image display device 6. When the receiving device 5 is attached, the cradle 6a acquires the image data from the memory of the receiving device 5, the received electric field strength information associated with the image data, and the associated information such as the time information and the identification information of the capsule endoscope 3, and the like, and transfers the acquired various sorts of information to the image display device 6. The operation input device 6b accepts the user input. This allows the user to observe the organism part such as the esophagus, the stomach, the small intestine, the large intestine, and so on and diagnose the subject 2 while operating the operation input device 6b and watching the image of the inside of the subject 2 displayed at the image display device 6.

Next, the configuration of the receiving device 5 illustrated in FIG. 1 will be described. FIG. 3 is a block diagram illustrating the configuration of the receiving device 5 illustrated in FIG. 1.

As illustrated in FIG. 3, the receiving device 5 has the receiving antennas 40a to 40h described above, an antenna switchover selection switching unit 49 for alternatively switching the receiving antennas 40a to 40h, a transceiving circuit 50 for applying the process such as demodulation to the wireless signal received via any one of the receiving antennas 40a to 40h selected by the antenna switchover selection switching unit 49, a signal processing circuit 51 for performing the signal processing for extracting the image data and the like from the wireless signal outputted from the transceiving circuit 50, a received electric field strength detecting unit 52 for detecting the received electric field strength based on the strength of the wireless signal outputted from the transceiving circuit 50, an antenna power switchover selecting unit 53 for alternatively switching the receiving antennas 40a to 40h to supply power to any one of the receiving antennas 40a to 40h, a display unit 54 for displaying the image corresponding to the image data received from the capsule endoscope 3, an operating unit 55 for making an instruction operation, a storage unit 56 for storing various sorts of information including the image data received from the capsule endoscope 3, an I/F unit 57 for communicating with the image display device 6 interactively via the cradle 6a, a power supply unit 58 for supplying power to respective units of the receiving device 5, and a control unit 59 for controlling the operation of the receiving device 5.

The receiving antenna 40a has an antenna unit 41a, an active circuit 42a, and an antenna cable 43a. The antenna unit 41a is configured with, for example, an open antenna or a loop antenna, and receives the wireless signal transmitted from the capsule endoscope 3. The active circuit 42a is connected to the antenna unit 41a for matching the impedance of the antenna unit 41a, amplifying and/or attenuating the received wireless signal, and so on. The antenna cable 43a is configured with a coaxial cable, one end of which is electrically connected to the active circuit 42a and the other end of which is electrically connected to the antenna switchover selection switching unit 49 and the antenna power switchover selecting unit 53, respectively. The antenna cable 43a transmits the wireless signal received by the antenna unit 41a to the receiving device 5 and transfers the power supplied from the receiving device 5 to the active circuit 42a. It is noted that, since each of the receiving antennas 40b to 40h has the same configuration as the receiving antenna 40a, the description thereof is omitted.

The antenna switchover selection switching unit 49 is configured with a mechanical switch, a semiconductor switch, and the like. The antenna switchover selection switching unit 49 is electrically connected to each of the receiving antennas 40a to 40h via a capacitor C1. When a switching signal S1 for switching the receiving antennas 40a to 40h for receiving the wireless signal from the control unit 59 is inputted, the antenna switchover selection switching unit 49 selects the receiving antenna 40 instructed by the switching signal S1, and outputs the wireless signal received via the selected receiving antennas 40a to 40h to the transceiving circuit 50. It is noted that each capacitor connected to each of the receiving antennas 40a to 40h has the same capacitance as the capacitor C1.

The transceiving circuit 50 applies a specified operation such as demodulation, amplification, and so on to the wireless signal received via the receiving antenna 40 (40a to 40h) selected by the antenna switchover selection switching unit 49 and outputs it to the signal processing circuit 51 and the received electric field strength detecting unit 52, respectively.

The signal processing circuit 51 extracts the image data from the wireless signal inputted from the transceiving circuit 50, and applies a specified process such as various sorts of image processing, A/D conversion processing, and so on to the extracted image data and outputs it to the control unit 59.

The received electric field strength detecting unit 52 detects the received electric field strength depending on the strength of the wireless signal inputted from the transceiving circuit 50 and outputs, to the control unit 59, the received electric field strength signal (RSSI: Received Signal Strength Indicator) corresponding to the strength of the wireless signal inputted from the transceiving circuit 50.

The antenna power switchover selecting unit 53 is electrically connected to each of the receiving antennas 40a to 40h via a coil L1. The antenna power switchover selecting unit 53 supplies power to the receiving antennas 40a to 40h selected by the antenna switchover selection switching unit 49 via the antenna cable 43 (43a to 43h). The antenna power switchover selecting unit 53 has a power switchover selection switching unit 531 and an abnormality detecting unit 532. It is noted that the coil connected to each of the receiving antennas 40a to 40h has the same electric properties as the coil L1.

The power switchover selection switching unit 531 is configured with a mechanical switch, a semiconductor switch, and the like. When a selection signal S2 for selecting the receiving antennas 40a to 40h to be supplied with power from the control unit 59 is inputted, the power switchover selection switching unit 531 selects the receiving antennas 40a to 40h as instructed by the selection signal S2 and supplies power only to the selected receiving antenna 40a to 40h.

When an abnormality occurs in the receiving antenna 40a to 40h to be supplied with power, the abnormality detecting unit 532 outputs, to the control unit 59, an abnormality signal indicating the occurrence of the abnormality in the receiving antenna 40a to 40h to be supplied with power.

The display unit 54 is configured with a display panel of the liquid crystal, the organic EL (Electro Luminescence), and the like. The display unit 54 displays various sorts of information such as the image corresponding to the image data taken by the capsule endoscope 3, the operation state of the receiving device 5, the patient information for the subject 2, the examination date and time, and so on.

The operating unit 55 is able to input the instruction signal such as for instructing the capturing period of the capsule endoscope 3 to be changed. In response to the instruction signal inputted from the operating unit 55, the signal processing circuit 51 sends the instruction signal to the transceiving circuit 50 and the transceiving circuit 50 modulates the instruction signal to transmit it from the receiving antennas 40a to 40h. The signals transmitted from the receiving antennas 40a to 40h are received by an antenna 39 and demodulated by the transceiving circuit 37, and the circuit board 36 operates for changing the capturing period, for example, in response to the instruction signal.

The storage unit 56 is configured with a semiconductor memory such as a flash memory, a RAM (Random Access Memory), and the like fixedly provided inside the receiving device 5. The storage unit 56 has theoretical electric field strength data 561 for the estimation process of the position and orientation, at which the image data has been taken, of the capsule endoscope 3 inside the subject 2. The theoretical electric field strength data 561 is the theoretical value data of the received electric field strength of the wireless signal received by each of the receiving antennas 40a to 40h depending on the position and orientation of the capsule endoscope 3 in the subject 2. Further, the storage unit 56 stores the image data taken by the capsule endoscope 3 and various sorts of information associated with that image data, such as the estimated position and orientation information of the capsule endoscope 3, the received electric field strength information, and the identification information for identifying the receiving antenna which has received the wireless signal, and so on. Further, the storage unit 56 stores various programs and the like that is executed by the receiving device 5. It is noted that the storage unit 56 may be provided with the function of a recording medium interface for storing the information to a recording medium such as a memory card and the like from the external unit while reading out the information stored in the recording medium.

The I/F unit 57 has the function as the communication interface and communicates bi-directionally with the image display device 6 via the cradle 6a.

The power supply unit 58 is configured with a battery that is removable from the receiving device 5 and a switch unit that switches a turn-on/off state. The power supply unit 58 supplies a driving power necessary for each component of the receiving device 5 in the turn-on state, while stops supplying the driving power to each component of the receiving device 5 in the turn-off state.

The control unit 59 is configured with a CPU (Central Processing Unit) and the like. The control unit 59 reads out the program from the storage unit 56 to execute it and transfers the instruction, the data, and so on to each unit of the receiving device 5 to control the operation of the receiving device 5 in an integrated manner. The control unit 59 has a selection control unit 591, an abnormality information adding unit 592, an electric field strength comparing unit 593, and a position determination unit 594.

The selection control unit 591 selects one receiving antenna 40 for receiving the wireless signal transmitted from the capsule endoscope 3 and controls to supply power only to the selected receiving antenna 40a to 40h. Specifically, the selection control unit 591 selects one receiving antenna 40 for receiving the wireless signal transmitted from the capsule endoscope 3 based on the received electric field strength of each of the receiving antennas 40a to 40h detected by the received electric field strength detecting unit 52, and controls to supply power only to the selected antenna 40a to 40h. The selection control unit 591 drives the antenna switchover selection switching unit 49 for every specified timing such as for every 100 msec, and sequentially selects one receiving antenna 40a to 40h for receiving the wireless signal from the receiving antennas 40a to 40h and causes the received electric field strength detecting unit 52 to detect the received electric field strength.

When the abnormality detecting unit 532 detects an abnormality at any one of the receiving antennas 40a to 40h, the abnormality information adding unit 592 adds, to the wireless signal received by the receiving antenna 40, abnormality information indicating the occurrence of the abnormality at any one of the receiving antennas 40a to 40h and outputs it to the storage unit 56. Specifically, the abnormality information adding unit 592 adds the abnormality information (flag) to the image data for which the signal processing circuit 51 has applied the signal processing to the wireless signal received by the receiving antennas 40a to 40h, and outputs it to the storage unit 56.

The electric field strength comparing unit 593 calculates the residual sum of squares between the received electric field strength of the wireless signal received by each of the receiving antennas 40a to 40h and the theoretical electric field strength stored in the storage unit 56 for each position and orientation within the subject 2 at which the capsule endoscope 3 can be present inside the subject 2. The electric field strength comparing unit 593 may calculate and compare the sum of the absolute residuals between the received electric field strength and the theoretical electric field strength in place of the residual sum of squares.

Based on the residual sum of squares or the sum of the absolute residuals calculated by the electric field strength comparing unit 593, the position determination unit 594 determines the position and orientation of the capsule endoscope 3 at which the image data has been taken. The position determination unit 594 determines the region and orientation having the smallest residual sum of squares as the position and orientation of the capsule endoscope 3 at which the image data has been taken.

In the first example, the receiving device 5 has the storage unit 56 for storing the theoretical electric field strength data 561, the electric field strength comparing unit 593 for calculating the residual sum of squares of the received electric field strength and the theoretical electric field strength, and the position determination unit 594 for determining the position and orientation of the capsule endoscope 3 based on the residual sum of squares calculated by the electric field strength comparing unit 593, and calculates with these elements the position and orientation of the image data taken by the capsule endoscope 3. Described below in detail will be the estimation process of the position and orientation of the capsule endoscope 3 in the receiving device 5 of the first example.

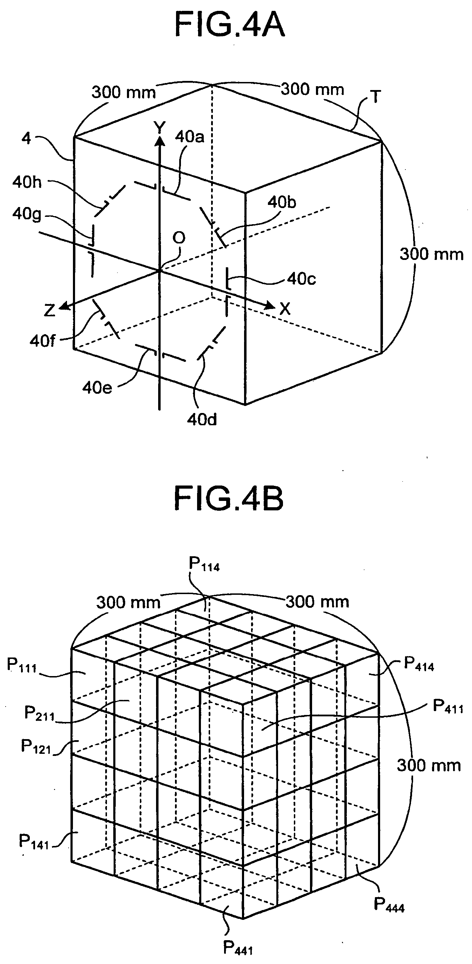

First, described will be the calculation process of the theoretical electric field strength data 561 to be pre-stored in the storage unit 56. A specified possible occurrence region T where the capsule endoscope 3 can be present within the subject 2 into which the capsule endoscope 3 is introduced is initially set according to the purpose of the examination, the diagnosis, and the like. This possible occurrence region T is set depending on the size of the body of the subject 2, which will be a region of a cube of 300 mm $\times$ 300 mm $\times$ 300 mm as illustrated in FIG. 4A, for example. The possible occurrence region T is set so that the sheet-shaped surface of the receiving antenna unit 4 matches one of the border planes. In the case illustrated in FIG. 4A, the receiving antenna unit 4 is provided on the XY plane that is one of the border planes of the possible occurrence region T.

The possible occurrence region of the capsule endoscope 3 is divided into a plurality of subregions according to the desired accuracy. FIG. 4B illustrates a case where it is divided into four regions in each axis direction with respect to the orthogonal coordinate system XYZ having three axes (X axis, Y axis, Z axis) that are parallel to any one of the edges of the possible occurrence region T and are orthogonal to each other, where the origin is assumed to the center of the border plane on which the receiving antenna unit 4 is located. In this case, the possible occurrence region T is divided into 64 (= $4 \times 4 \times 4$) subregions. The subregions are labeled with $P_{111}$, $P_{112}$, $P_{113}$, $P_{114}$, $P_{121}$, $P_{122}$, ..., $P_{211}$, $P_{212}$, ..., $P_{444}$. It is noted that, when the capsule endoscope 3 is present in a subregion $P_{ijk}$, it is assumed the capsule endoscope 3 being located at the center $G_{xyz}$ of the subregion $P_{ijk}$.

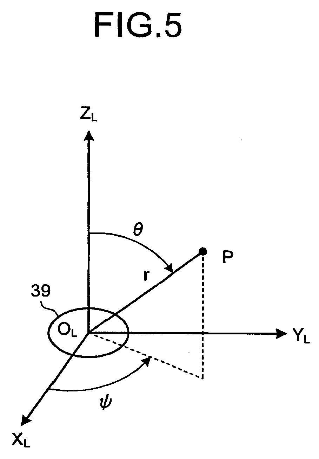

In the following description, as illustrated in FIG. 5, the center of gravity of the circle loop antenna 39 disposed in the capsule endoscope 3 is defined as the origin ($O_L$), and the orthogonal coordinate system $X_L Y_L Z_L$ will be considered where the normal line direction of the opening plane of the circle loop is defined as the $Z_L$ axis. In the orthogonal coordinate system $X_L Y_L Z_L$, the polar coordinate components of the electromagnetic field that is formed at a particular position P by the current flowing in the antenna 39 are represented by the following equations.

$$H_r = (IS/2\pi)(jk/r^2 + 1/r^3)\exp(-jkr)\cos\theta$$

$$H_\theta = (IS/4\pi)(-k^2/r + jk/r^2 + 1/r^3)\exp(-jkr)\sin\theta \quad (1)$$

$$E_\psi = -(j\omega\mu IS/4\pi)(jk/r + 1/r^2)\exp(-jkr)\sin\theta$$

Here, $H_r$ and $H_\theta$ denote the magnetic field components, $E_\psi$ denotes the electric field component, and I and S denote the current flowing in the antenna 39 and the region of the opening region of the circle loop of the antenna 39. Further, $k = \omega(\varepsilon\mu)^{1/2}$ ($\varepsilon$ is the dielectric constant, $\mu$ is the magnetic permeability) denotes the number of the waves, and j represents the unit of the imaginary number. Here, in Equations (1), the term of $r^{-1}$ denotes the radiation electromagnetic field, the term of $r^{-2}$ denotes the induction electromagnetic field, and the term of $r^{-3}$ denotes the static electromagnetic field.

When the frequency of the electromagnetic field generated by the antenna 39 disposed within the capsule endoscope 3 is high and the capsule endoscope 3 and each receiving antenna 40 (40a to 40h) attached to the body surface of the subject 2 are sufficiently distant, the component of the radiation electromagnetic field is the greatest in the electromagnetic field (the electromagnetic wave) reaching the receiving antenna 40 (40a to 40h). Thus, the components of the static electromagnetic field and the induction electromagnetic field are smaller than the component of the radiation electromagnetic field and thus can be ignored. Therefore, Equations (1) can be expressed as the following Equations (2).

$$H_r = 0$$

$$H_\theta = (IS/4\pi)(-k^2/r)\exp(-jkr)\sin\theta \tag{2}$$

$$E_\psi = -(j\omega\mu IS/4\pi)(jk/r)\exp(-jkr)\sin\theta$$

Assuming that the receiving antenna 40 attached to the body surface of the subject 2 is the electric field detecting antenna for detecting the electric field, the necessary equation for detecting it in Equations (2) will be the electric field $E_\psi$. Therefore, the instantaneous value of the electric field can be derived by using the alternating current theory, that is, multiplying both sides of the electric field $E_\psi$ of Equations (2) by $\exp(j\omega t)$ to extract the real number part.

$$E_\psi \exp(j\omega t)$$

$$= -(j\omega\mu IS/4\pi)(jk/r)\exp(-jkr)\sin\theta\exp(j\omega t)$$

$$= (\omega\mu ISk/4\pi r)(\cos U + j\sin U)\sin\theta \tag{3}$$

Here, U = $\omega$t - kr. Then, the real number part of Equation (3) is extracted to have the following instantaneous value $E'_\psi$ of the electric field.

$$E'_\psi = (\omega\mu ISk/4\pi r)\cos U \sin\theta \tag{4}$$

Further, when Equation (4) is represented in the orthogonal coordinate system $X_L Y_L Z_L$, the components $E_{Lx}$, $E_{Ly}$, and $E_{Lz}$ will be as follows.

$$E_{Lx} = E'_\psi \sin\psi = (\omega\mu ISk/4\pi r^2)\cos U \cdot (-y_L)$$

$$E_{Ly} = E'_\psi \cos\psi = (\omega\mu ISk/4\pi r^2)\cos U \cdot x_L \tag{5}$$

$$E_{Lz} = 0$$

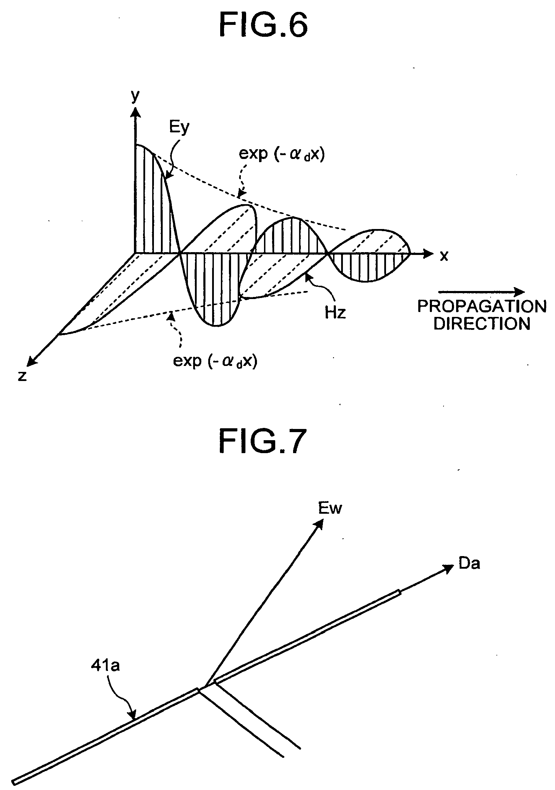

When the electromagnetic wave travels in the medium, the characteristics (the electric conductivity and the like) of the medium causes the energy of the electromagnetic wave to be absorbed in the medium through which the electromagnetic wave travels, as illustrated in FIG. 6. The electromagnetic wave attenuates exponentially at an attenuation factor $\alpha_d$ as it propagates in the x direction, which can be expressed by the following Equations (6).

$$A_r = \exp(-\alpha_d x) \tag{6}$$

$$\alpha_d = (\omega^2 \varepsilon\mu/2)^{1/2}[(1 + \kappa^2/(\omega^2\varepsilon^2))^{1/2} - 1]^{1/2}$$

Here, $\varepsilon = \varepsilon_o \varepsilon_r$ ($\varepsilon_o$: the dielectric constant of the vacuum, $\varepsilon_r$: the relative dielectric constant), $\mu = \mu_o \mu_r$ ($\mu_o$: the magnetic permeability of the vacuum, $\mu_r$: the relative magnetic permeability), $\omega$ is the angular frequency, and $\kappa$ is the electric conductivity.

Therefore, respective components $E_{Lx}$, $E_{Ly}$, and $E_{Lz}$ of the orthogonal coordinate system $X_L Y_L Z_L$ of the instantaneous value $E_L$ of the electric field at the time when the characteristics in the organism is taken into consideration are as follows.

$$E_{Lx} = A_r E'_\psi \sin\psi = \exp(-\alpha_d\ r)\ (\omega\mu I S k/4\pi r^2)\ \cos U \cdot (-y_L)$$

$$E_{Ly} = A_r E'_\psi \cos\psi = \exp(-\alpha_d\ r)\ (\omega\mu I S k/4\pi r^2)\ \cos U \cdot x_L \qquad (7)$$

$$E_{Lz} = 0$$

Further, in the coordinate system $X_L Y_L Z_L$ with respect to the antenna 39 of the capsule endoscope 3, the equation for converting the position P ($X_L$, $Y_L$, $Z_L$) into the coordinate system $X_W Y_W Z_W$ whose origin is the center (O in FIG. 4A) of the receiving antenna unit 4 attached to the subject 2 is:

$$\begin{pmatrix} x_{LP} \\ y_{LP} \\ z_{LP} \end{pmatrix} = R^{-1}\left[ \begin{pmatrix} x_{WP} \\ y_{WP} \\ z_{WP} \end{pmatrix} - \begin{pmatrix} x_{WG} \\ y_{WG} \\ z_{WG} \end{pmatrix} \right] = \begin{pmatrix} R_{00} & R_{01} & R_{02} \\ R_{10} & R_{11} & R_{12} \\ R_{20} & R_{21} & R_{22} \end{pmatrix}\left[ \begin{pmatrix} x_{WP} \\ y_{WP} \\ z_{WP} \end{pmatrix} - \begin{pmatrix} x_{WG} \\ y_{WG} \\ z_{WG} \end{pmatrix} \right] \qquad (8)$$

Here, ($x_{WP}$, $y_{WP}$, $z_{WP}$) and ($x_{WG}$, $y_{WG}$, $z_{WG}$) represent the position P in the coordinate system $X_W Y_W Z_W$ and the position G of the antenna 39, respectively. Further, the right side R of Equation (8) represents the rotation matrix of the coordinate system $X_W Y_W Z_W$ and the coordinate system $X_L Y_L Z_L$, and can be derived by the following equation.

$$\begin{pmatrix} R_{00} & R_{10} & R_{20} \\ R_{01} & R_{11} & R_{21} \\ R_{02} & R_{12} & R_{22} \end{pmatrix} = \begin{pmatrix} \cos\alpha\cos\beta & -\sin\alpha & \cos\alpha\sin\beta \\ \sin\alpha\cos\beta & \cos\alpha & \sin\alpha\sin\beta \\ -\sin\beta & 0 & \cos\beta \end{pmatrix} \qquad (9)$$

Here, $\alpha$ is the rotation angle around the Z axis and $\beta$ is the rotation angle around the Y axis.

Therefore, the electric field $E_w$ of some particular position P ($x_{WP}$, $y_{WP}$, $z_{WP}$) in the coordinate system $X_W Y_W Z_W$ whose origin is the center (O in FIG. 4A) of the receiving antenna unit 4 attached to the subject 2 is:

$$Ew = \begin{pmatrix} E_{Wx} \\ E_{Wy} \\ E_{Wz} \end{pmatrix} = R\begin{pmatrix} E_{Lx} \\ E_{Ly} \\ E_{Lz} \end{pmatrix} = \begin{pmatrix} R_{00} & R_{10} & R_{20} \\ R_{01} & R_{11} & R_{21} \\ R_{02} & R_{12} & R_{22} \end{pmatrix}\begin{pmatrix} E_{Lx} \\ E_{Ly} \\ E_{Lz} \end{pmatrix} \qquad (10)$$

Then, Equations (7) to (9) are substituted in Equation (10) resulting in the following equation of the electric field $E_w$ (11).

$$\begin{pmatrix} E_{Wx} \\ E_{Wy} \\ E_{Wz} \end{pmatrix} = \frac{k_1}{r^2}e^{-\alpha_d r}\begin{pmatrix} 0 & (z_{WP}-z_{WG}) & -(y_{WP}-y_{WG}) \\ -(z_{WP}-z_{WG}) & 0 & (x_{WP}-x_{WG}) \\ (y_{WP}-y_{WG}) & -(x_{WP}-x_{WG}) & 0 \end{pmatrix}\begin{pmatrix} g_x \\ g_y \\ g_z \end{pmatrix} \qquad (11)$$

Here, $k_1$ is a constant, the vector ($g_x$, $g_y$, $g_z$) denotes the orientation g of the antenna 39. In the first example, the orientation ($g_x$, $g_y$, $g_z$) of the antenna 39 is preset together with the position of the capsule endoscope 3 to calculate the theoretical electric field strength of each receiving antenna 40 when the capsule endoscope 3 is located at a specified region and at a specified orientation. The orientation of the antenna 39 may be set by 1° pitch from the horizontal axis and the vertical axis, for example, according to the desired accuracy.

Further, the electromotive force $V_{ta}$ detected when the electric field $E_w$ generated by the antenna 39 is received by the receiving antenna 40a of the receiving antenna unit 4 can be calculated by using the following equation in a use of the inner product of the electric field $E_w$ and the vector $D_a = (D_{xa}, D_{ya}, D_{za})$ (see FIG. 7) representing the orientation of the receiving antenna 40a (the antenna unit 41a) of the receiving antenna unit 4 in the coordinate system with respect to the subject 2.

$$V_{ta} = k_2 \ (E_{Wx}D_{xa} + E_{Wy}D_{ya} + E_{Wz}D_{za}) \hspace{2cm} (12)$$

Here, $k_2$ is a constant. For each of the plurality of receiving antennas of the receiving antenna unit 4 provided to the body of the subject 2, the similar is applied to derive the electromotive forces $V_{tb}$, ... , $V_{th}$ when the receptions are made at the receiving antenna 40b to the receiving antenna 40h.

In such a manner as described above, the theoretical electric field strength $V_{ti}$ received by each receiving antenna 40 is calculated and stored in the storage unit 56 as the theoretical electric field strength data 561 for each center position G in the divided region.

The electric field strength comparing unit 593 calculates the residual sum of squares between the received electric strength received by each receiving antenna 40 and the theoretical electric field strength stored in the storage unit 56 as the theoretical electric field strength data 561 calculated as described above for each orientation g of the antenna 39 for the center position G of each region where the capsule endoscope 3 can be present. Assuming that the electric field strength $V_{mi}$ ("i" represents the number of the receiving antenna, i = a to h in the present example) received by the receiving antenna 40, the residual sum of squares S can be calculated by the following equation.

$$S = \sum_{i=a}^{h}(V_{ti} - V_{mi})^2$$
$$= (V_{ta} - V_{ma})^2 + (V_{tb} - V_{mb})^2 + \cdots + (V_{th} - V_{mh})^2 \hspace{2cm} (13)$$

In the first example, as described above, the electric field strength comparing unit 593 calculates the residual sum of squares between the received electric field strength $V_{mi}$ received by each receiving antenna 40 and the theoretical electric field strength $V_{ti}$ stored in the storage unit 56 as the theoretical electric field strength data 561 calculated as described above for each orientation g of the antenna 39 for the center position G of each region where the capsule endoscope 3 can be present, so that the same number of the CPUs as the total number of the center positions G for estimation (alternatively, it may be the factor of the total number of the center positions G for estimation, or the number less than or equal to the factor) can be used as the electric field strength comparing unit 593 at the same time for the estimation process, which allows for the faster estimation process of the position and orientation of the capsule endoscope 3.

The position determination unit 594 determines, as the position and orientation of the capsule endoscope 3, the center position G of the capsule endoscope 3 and the orientation g of the antenna 39 having the smallest one of the residual sum of squares S calculated by the electric field strength comparing unit 593 as described above.

In the first example, the region where the capsule endoscope 3 can be present is divided into a plurality of subregions and the theoretical electric field strength $V_{ti}$ depending on the orientation of the capsule endoscope 3 for each divided region is stored in advance, so that the processing load for calculating the theoretical electric field strength $V_{ti}$ can be reduced. Further, the position and orientation of the capsule endoscope 3 at which the image data has been taken is determined based on the numeral value that can be obtained by simple calculation process of the residual sum of squares between the stored theoretical electric field strength $V_{ti}$ and the received electric field $V_{mi}$ that has been actually received by the receiving antenna 40, which allows for the faster position estimation process.

Furthermore, in the first example, the sheet-shaped receiving antenna unit 4 on which a plurality of receiving antennas 40 are provided, so that it is not necessary to adjust the position of each receiving antenna 40 for every examination. Moreover, the receiving antenna unit 4 on which the position of each receiving antenna 40 is determined in advance is used, so that the problem of reduction in accuracy in the estimation process of the position and orientation of the capsule endoscope 3 that would otherwise be caused by the position shift of each receiving antenna 40 can be advantageously avoided.

Although the position detecting apparatus for performing the estimation process of the position and orientation of the capsule endoscope 3 has been described in the first example, the apparatus may estimate either one of the position and the orientation of the capsule endoscope 3. Further, the receiving device 5 includes the storage unit 56 for storing the theoretical electric field strength data 561, the electric field strength comparing unit 593, and the position determination unit 594, and the position and orientation of the capsule endoscope 3 is estimated in the receiving device 5 in the first example. However, the image display device 6 of the capsule endoscope system 1 may store the theoretical electric field strength, include the electric field strength comparing unit and the position determination unit, receive the image data transmitted from the receiving device, and perform the calculation similarly to the above to estimate the position and orientation of the capsule endoscope at which the image data has been taken.

(First Embodiment)

In the first example, the estimation process of the position and orientation of the capsule endoscope is made by calculating in parallel the residual sum of squares between the theoretical electric field strength and the received electric field strength with respect to all the set directions (alternatively, the reduced directions) in all the regions (alternatively, the reduced regions for simplification) divided as the position P where the capsule endoscope can be present. In contrast, a first embodiment divides it into hierarchies with two or more steps and determines the position and orientation of the capsule endoscope at which the image data has been taken.

In the first embodiment below, described will be a case where the estimation of the position and orientation of the capsule endoscope is divided into two steps. First, similarly to the first example, the possible occurrence region T where the capsule endoscope 3 can be present is set in the subject 2 into which the capsule endoscope 3 is introduced, according to the purpose of examination, diagnosis, and the like. For example, it may be the region of a cube of 300 mm $\times$ 300 mm $\times$ 300 mm as illustrated in FIG. 4A. The possible occurrence region T is set so that the sheet-shaped surface of the receiving antenna unit 4 matches one of the border planes. In the case illustrated in FIG. 4A, the receiving antenna unit 4 is provided on the XY plane that is one of the border planes of the possible occurrence region T.

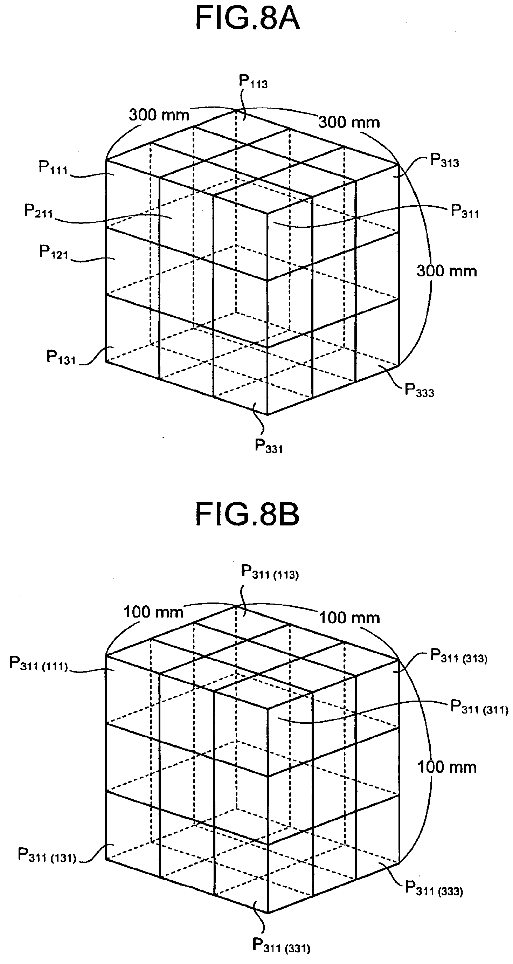

The possible occurrence region T of the capsule endoscope 3 is divided into a plurality of subregions according to the desired accuracy. FIG. 8A illustrates a case where it is divided into three regions in each axis direction with respect to the orthogonal coordinate system XYZ having three axes (X axis, Y axis, Z axis) that are parallel to any one of the edges of the possible occurrence region T and are orthogonal to each other, where the origin is assumed to the center of the border plane on which the receiving antenna unit 4 is located. In this case, the possible occurrence region T is divided into 27 (= 3 $\times$ 3 $\times$ 3) subregions. The subregions are labeled with $P_{111}$, $P_{112}$, $P_{113}$, $P_{121}$, $P_{122}$, ..., $P_{133}$, $P_{211}$, $P_{212}$, ..., $P_{333}$. It is noted that, when the capsule endoscope 3 is present in a subregion $P_{ijk}$, it is assumed to be located at the center $G_{xyz}$ of the subregion $P_{ijk}$.

FIG. 8A is a schematic diagram in which the possible occurrence region T of the capsule endoscope 3 is divided into three regions in each of the x, y, and z axis directions. FIG. 8B is a schematic diagram in which one of the divided regions in FIG. 8A is further divided into three regions in each of the x, y, and z axis directions.

The position detecting apparatus performs, as a first estimation step, an estimation process of the position and orientation for the regions resulted after the possible occurrence region T (300 mm $\times$ 300 mm $\times$ 300 mm) where the capsule endoscope 3 can be present within the subject 2 is divided into three regions for each of the x, y, and z axis directions. As illustrated in FIG. 8A, for each center position G of the regions labeled with $P_{111}$, $P_{112}$, $P_{113}$, $P_{121}$, $P_{122}$, ..., $P_{133}$, $P_{211}$, $P_{212}$, ..., $P_{333}$, the electric field strength comparing unit 593 calculates the residual sum of squares between the received electric field strength received by each receiving antenna 40 and the theoretical electric field strength stored as the theoretical electric field strength data 561 in the storage unit 56 for each orientation g of the antenna 39. Since a purpose of the first estimation step is to approximately specify the position and orientation, the orientation g of the antenna 39 in the estimation process is reduced to one, or is significantly reduced (for example, by 10° pitch from the horizontal axis and the vertical axis).

The position determination unit 594 determines, as the position and orientation of the first step, the center position G of the region of the capsule endoscope 3 and the orientation g of the antenna 39 which has the smallest one of the residual sums of squares S calculated by the electric field strength comparing unit 593.

As a second estimation step, another estimation process of the position and orientation is made for the regions resulted after the region including the position G of the capsule endoscope 3 determined by the position determination unit 594 at the first estimation step is further divided into three regions for each of the x, y, and z axis directions (27 regions in total).

For example, as the first estimation step, it is assumed that the position determination unit 594 selects the position $P_{311}$ illustrated in FIG. 8A as the position and orientation of the capsule endoscope 3. FIG. 8B illustrates that the position $P_{311}$ is divided into three regions for each axis direction with respect to the orthogonal coordinate system XYZ having three axes (X axis, Y axis, Z axis) that are parallel to one of the edges of the position $P_{311}$ and orthogonal to each other. In this case, the position $P_{311}$ is further divided into 27 (= 3 $\times$ 3 $\times$ 3) subregions. The subregions are labeled with $P_{311(111)}$, $P_{311(112)}$, $P_{311(113)}$, $P_{311(121)}$, $P_{311(122)}$, ..., $P_{311(133)}$, $P_{311(211)}$, $P_{311(212)}$, ..., $P_{311(333)}$. The electric field strength comparing unit 593 calculates the residual sum of squares between the received electric field strength received by each receiving antenna 40 and the theoretical electric field strength stored as the theoretical electric field strength data 561 in the storage unit 56 for each orientation g of the antenna 39. In the second estimation step, the orientation g of the antenna 39 is estimated according to the desired accuracy. For example, it is applied for all the directions by 1° pitch from the horizontal axis and the vertical axis.

The position determination unit 594 determines, as the final position and orientation of the capsule endoscope 3 at which the image data has been taken, the position $P_{xyz(xyz)}$ of the capsule endoscope 3 and the orientation $g_n$ ($g_{nx}$, $g_{ny}$, $g_{nz}$) of the antenna 39 which has the smallest one of the residual sums of squares $S_{xyz(xyz)n}$ calculated by the electric field strength comparing unit 593.

It is noted that, as described above, even if the estimation process is performed with the division of two steps of hierarchy, it is necessary for the storage unit 56 to store the theoretical electric field strength data 561 received by each receiving antenna 40 by each orientation g of the antenna 39 (by 1° pitch from the horizontal axis and the vertical axis) in the region position $P_{xyz(xyz)}$ resulted after the possible occurrence region T (300 mm × 300 mm × 300 mm) where the capsule endoscope 3 can be present is divided into nine regions for each of the x, y, and z axis directions.

In the first embodiment, the position and orientation of the capsule endoscope 3 at which the image data has been taken is divided into two steps of hierarchy, the approximate position and orientation of the capsule endoscope 3 is determined in the first estimation step, and the second estimation process is further applied to the limited regions, so that the amount of processing can be reduced compared to the case where the estimation process is applied at the same time for the regions of a similar size. This allows for much faster position estimation process.

It is noted that, although the example in which the position and orientation of the capsule endoscope 3 is divided into two steps of hierarchy for the estimation process has been described in the first embodiment, the estimation process may be performed with the division of three or more steps of hierarchy, because two or more steps can allow for the reduced amount of the estimation processing. It is noted that, regarding the orientation of the capsule endoscope 3 (the orientation of the antenna 39), the estimation process may be performed at a desired accuracy at the first step, for example, by 1° pitch from the horizontal axis and the vertical axis for all the directions.

(Second Embodiment)

When the position and orientation of the capsule endoscope 3 at which the image data has been taken is determined as described in the first example and the first embodiment, there is a case where the correct position and orientation cannot be estimated due to the positional error of the receiving antenna, the noise, and so on. In a second embodiment, the position and direction of the capsule endoscope 3 is estimated based on the estimated position information of the image data taken at a previous timing and a subsequent timing.

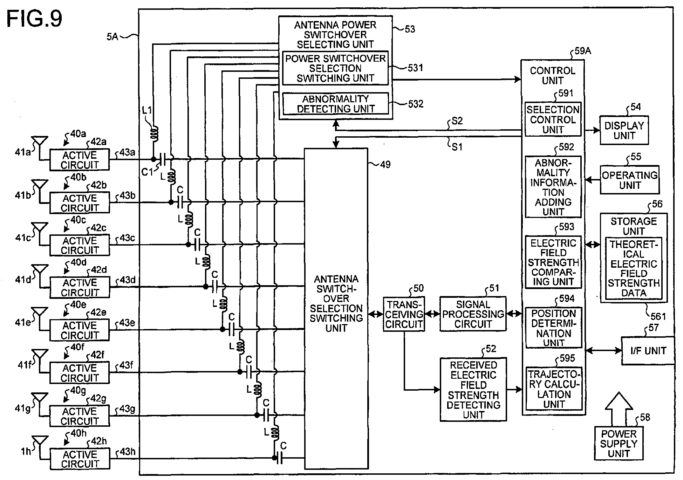

FIG. 9 is a block diagram illustrating a configuration of a receiving device 5A according to the second embodiment. The receiving device 5A includes a trajectory calculation unit 595 for calculating the distances between a plurality of candidate positions selected as the position of the capsule endoscope 3 by the position determination unit 594 and the previous and subsequent candidate positions, determining whether the distances are less than or equal to a specified value, and calculating the movement trajectory (path) of the capsule endoscope within the subject 2 using the distances which satisfy the condition.

In general, the movement of the capsule endoscope 3 is relatively small and the photographing interval is quite short. Thus, the capturing position of the image data that has been taken at a particular time is often substantially the same as or close to the capturing position of the image data that have been taken previously and subsequently with respect to the time when that image data has been taken.

In the second embodiment, the electric field strength comparing unit 593 calculates the residual sum of squares between the theoretical electric field strength and the received electric field strength similarly to the first example and the first embodiment. The position determination unit 594 selects the smallest i residual sums of squares (i is an arbitrary number, the present embodiment is described as i = 3) for the candidates of the position and orientation of the capsule endoscope at which the image data has been taken, and stores them into the storage unit 56. The trajectory calculation unit 595 calculates the movement trajectory of the capsule endoscope 3 within the subject 2 in taking into consideration of the estimated distance to a plurality of candidate positions of the image data taken at a previous timing and a subsequent timing. The position determination unit 594 determines the optimum position and orientation of the capsule endoscope based on the trajectory calculated by the trajectory calculation unit 595.

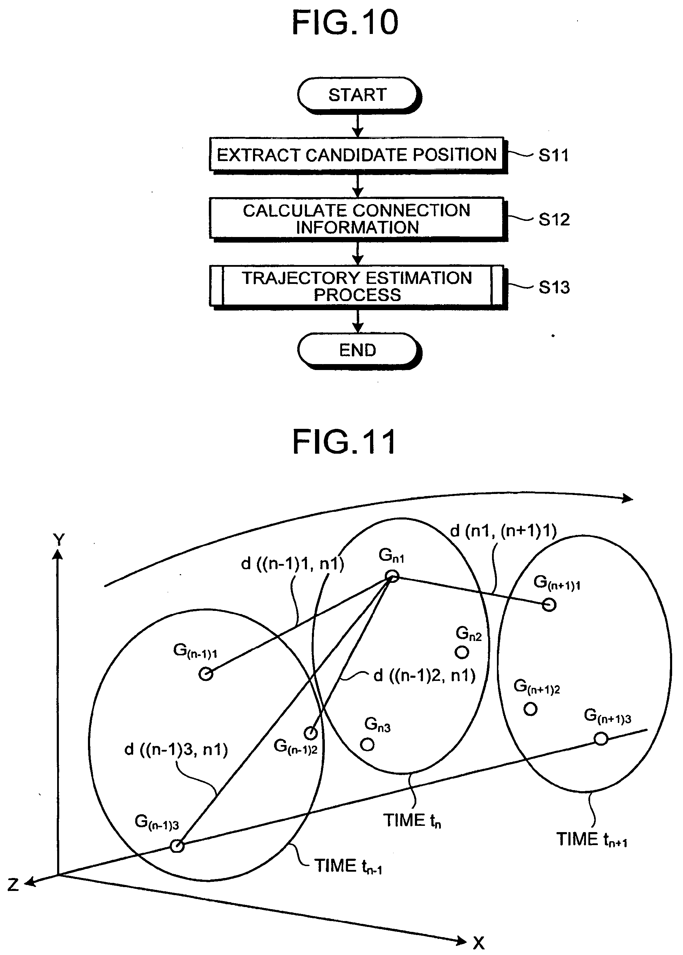

FIG. 10 is a flowchart illustrating the outline of the trajectory calculation process made by the trajectory calculation unit 595. The position determination unit 594 extracts the candidate position of the capturing position for each time calculated by the electric field strength comparing unit 593 (step S11). Specifically, the position determination unit 594 extracts, as the capturing position of the image data taken at each time, the three smallest residual sums of squares between the theoretical electric field strength and the received electric field strength in each position and orientation. Hereafter, the capturing position of the image data $D_m$, taken at the time $t_m$ (m = 1, 2, ..., n, ..., N) is assumed to be $G_{mi}$ (i = 1, 2, 3). FIG. 11 is a view schematically illustrating the candidate positions extracted at m = n-1, n, n+1.

Subsequently, the trajectory calculation unit 595 calculates the information of the connection to the candidate positions in the previous and subsequent image data with respect to the extracted candidate positions $G_{mi}$ (step S12). Here, the receiving device 5A pre-stores in the storage unit 56 the distance $r_d$ that the capsule endoscope 3 can move inside the subject 2 within one time interval depending on the time interval for the position estimation of the capsule endoscope 3.

13

The trajectory calculation unit 595 calculates the distances d((m-1)i, mj) between the candidate positions $G_{(m-1)i}$ of the image data $D_{m-1}$, $D_m$ at the neighboring time intervals $t_{m-1}$, $t_m$ and the $G_{mi}$ for all the combinations (m = 2, ..., N; i, j = 1, 2, 3), and compares the calculated distances with the movable distance $r_d$. As a result of the comparison, the candidate position $G_{(m-1)j}$ providing the distance d((m-1)i, mj) which is smaller than the movable distance $r_d$ and is the smallest is stored in the storage unit 56 as the connection information of the candidate position $G_{mi}$. It is noted that, if all the distances d((m-1)i, mj) are larger than the movable distance $r_d$ for a particular m, the trajectory calculation unit 595 does not store the connection information of the candidate position $Q_{mi}$.

Subsequently, the trajectory calculation unit 595 uses each candidate position $G_{mi}$ and the connection information of the candidate position to estimate the trajectory of the capsule endoscope 3 (step S13).

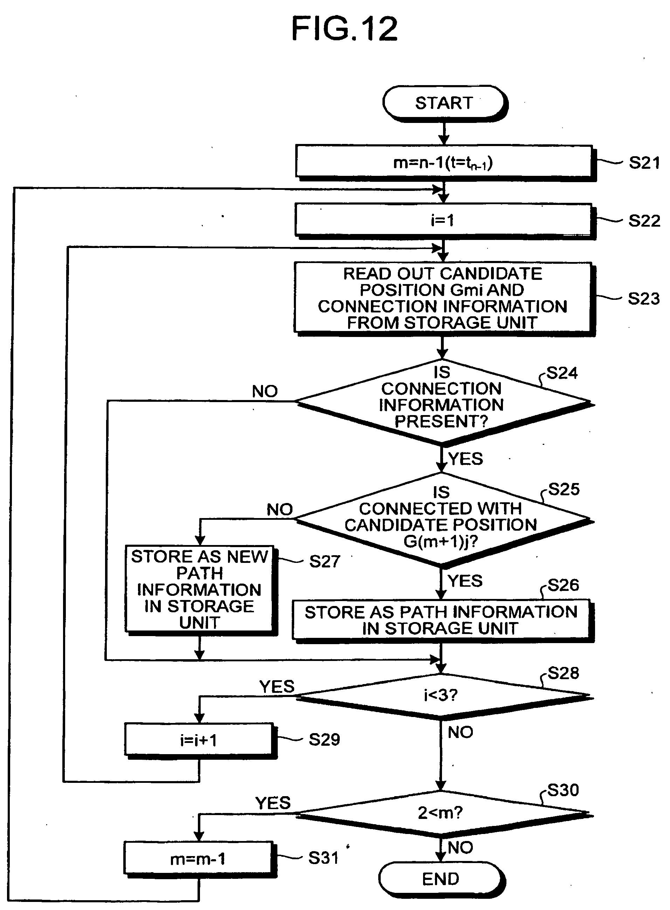

FIG. 12 is a flowchart illustrating an outline of the trajectory estimation process. In FIG. 12, the trajectory calculation unit 595 sets the time $t_{m-1}$ that is immediately before the last time $t_m$ (step S21).

Then, the trajectory calculation unit 595 sets the parameter, which indicates the label of the candidate position at the time $t_m$, to the initial value 1 (step S22).

Subsequently, the trajectory calculation unit 595 reads out the connection information of the candidate position $G_{mi}$ from the storage unit 56 at the time $t_m$ (step S23).

If there is connection information $G_{(m-1)j}$ in the candidate positions $G_{mi}$ (Yes in step S24), the trajectory calculation unit 595 determines whether or not the candidate position $G_{mi}$ is connected to the candidate position $G_{(m+1)j}$ at the time $t_{m+1}$ (step S25). If the candidate position $G_{mi}$ is connected to any one of the candidate positions $G_{(m+1)j}$ at the time $t_{m+1}$ (Yes in step S25), in other words, if the candidate position $G_{mi}$ is the connection information of any one of the candidate positions $G_{(m+1)j}$ at the time $t_{m+1}$, the trajectory calculation unit 595 stores the connected path information in the storage unit 56 (step S26).

On the other hand, if the candidate position $G_{mi}$ is not connected to any one of the candidate positions $G_{(m+1)j}$ at the time $t_{m+1}$ (No in step S25), in other words, if the candidate position $G_{mi}$ is not the connection information of any one of the candidate positions $G_{(m+1)j}$ at the time $t_{m+1}$, the trajectory calculation unit 595 stores new path information (the information indicating that the path is disconnected between the time $t_m$ and the time $t_{m+1}$) in the storage unit 56 (step S27).

After step S26 or S27, if the parameter i is less than three (Yes in step S28), the trajectory calculation unit 595 increments i by one to have i+1 (step S29) and returns to step S23.

After step S26 or S27, if the parameter i is not less than three (No in step S28), if the time parameter m is m > 2 (Yes in step S30), the trajectory calculation unit 595 decrements m by one to have m-1 (step S31) and returns to step S22. On the other hand, if m ≤ 2 (No in step S30), the trajectory calculation unit 595 finishes the trajectory estimation process (step S13 of FIG. 10).

In such a way, the trajectory calculation unit 595 calculates the trajectory and estimates the position of the capsule endoscope 3 for each time.

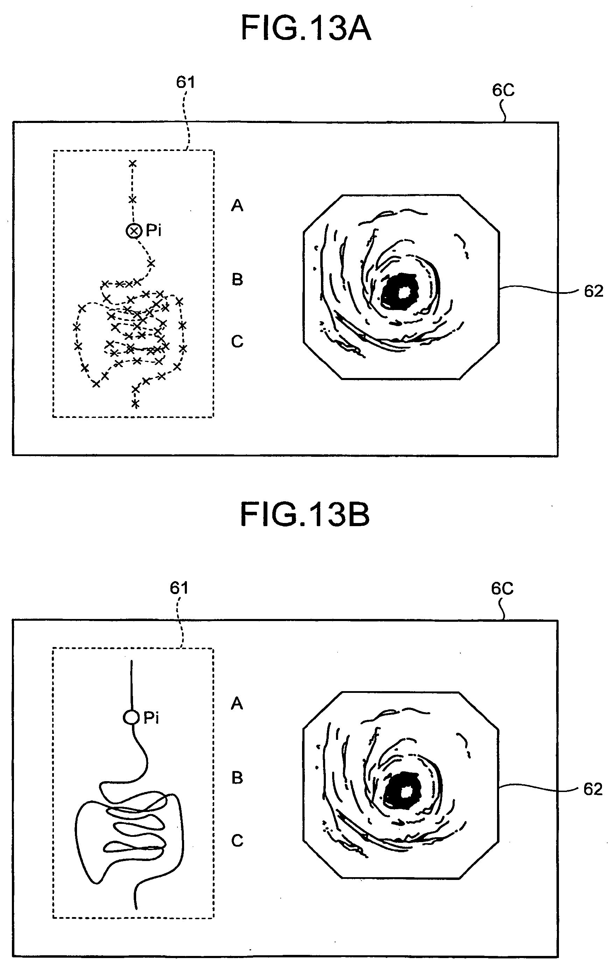

FIG. 13A and FIG. 13B are display examples on the monitor unit 6c of the image display device 6 that shows the trajectory of the capsule endoscope 3 within the subject 2 calculated by the receiving device 5A of the second embodiment. As illustrated in FIG. 13A, the monitor unit 6c includes a sub image region 61 in which the capturing positions of the capsule endoscope 3 within the subject 2 are connected by straight lines and the movement trajectory of the capsule endoscope 3 within the subject 2 is indicated, and a main image display region 62 in which the image data taken by the capsule endoscope 3 is displayed.

Further, the characters A, B, and C in the right of the sub image region 61 indicate the approximate positions of the organs in the body cavity. Specifically, the character A indicates the esophagus, the character B indicates the small intestine, and the character C indicates the large intestine. Further, the position $P_i$ indicates the position estimated as the capturing position of the image data which is displayed in the main image display region 62. Besides FIG. 13A in which the estimated capturing positions Pi are connected by the straight lines so that these are indicated as the trajectory, other display as illustrated in FIG. 13B, for example, may be employed in which the interpolation process such as the spline interpolation is applied to the neighboring capturing positions so that the capturing positions of the estimated capsule endoscope 3 are connected by smooth curves.

In the second embodiment, the position and orientation of the capsule endoscope 3 can be estimated without being affected by the noise and the like, so that the more accurate position and orientation of the capsule endoscope 3 can be derived. Further, the position and orientation of the capsule endoscope 3 is estimated and the movement trajectory of the capsule endoscope 3 inside the subject 2 is displayed on the image display device 6, so that it can be easily determined at which position in the body cavity the captured image has been taken, which allows for the efficient diagnosis. Further, there is a possibility that the obtained image is a lesion region, and when the further detailed endoscope examination is needed for that part, its position can be estimated in a high accuracy and thus the part can be approached smoothly in a short time, which allows for the efficient reexamination, treatment, and the like.

Industrial Applicability

A receiving device and a capsule endoscope system according to the present invention are useful for detecting a position of the capsule endoscope introduced into a subject, and particularly suitable for processing, by an image processing apparatus, image data captured by the capsule endoscope for diagnosis.

Reference Signs List

1 capsule endoscope system
2 subject
3 capsule endoscope
4 receiving antenna unit
5, 5A receiving device
6 image display device
6a cradle
6b operation input device
6c monitor unit
40a to 40h receiving antenna
41a to 41h antenna unit
42a to 42h active circuit
43a to 43h antenna cable
44 sheet
49 antenna switchover selection switching unit
50 transceiving circuit
51 signal processing circuit
52 received electric field strength detecting unit
53 antenna power switchover selecting unit
54 display unit
55 operating unit
56 storage unit
57 I/F unit
58 power supply unit
59 control unit
531 power switchover selection switching unit
532 abnormality detecting unit
591 selection control unit
592 abnormality information adding unit
593 electric field strength comparing unit
594 position determination unit
595 trajectory calculation unit

**Claims**

1.   A position detecting apparatus of a capsule endoscope (1) comprising:

a receiving antenna unit (4) configured to receive, by a plurality of receiving antennas (40a-40h), a wireless signal transmitted from a capsule endoscope (3) within a subject (2);
a storage unit (56) configured to store a theoretical electric field strength of the wireless signal received by each of the receiving antennas (40a-40h), the theoretical electric field strength depending on a position or a position and orientation of the capsule endoscope (3) in the subject (2);
an electric field strength comparing unit (593) configured to compare a specified value calculated by using a difference between a received electric field strength of the wireless signal received by each of the receiving antennas (40a-40h) and the theoretical electric field strength stored in the storage unit (56); and
a position determination unit (594) configured to determine a position and orientation of the capsule endoscope (3) where image data has been taken, based on a comparison result by the electric field strength comparing unit (593), **characterized in that**

the storage unit (56) is storing a theoretical electric field strength according to an orientation of the capsule endoscope (3) for each of a plurality of subregions ($P_{111}$, $P_{113}$, $P_{114}$, $P_{121}$, $P_{131}$, $P_{141}$, $P_{211}$, $P_{311}$, $P_{313}$, $P_{333}$, $P_{411}$, $P_{414}$, $P_{441}$, $P_{444}$) into which an occurrence region (T) within the subject (2) where the capsule endoscope (3) can be present is divided, and for each region into which each subregion is further divided,

the electric field strength comparing unit (593) is configured to calculate, as a first estimation step, a residual sum of squares between each of the theoretical electric field strengths stored in the storage unit (56) for each center position of the subregions ($P_{111}$, $P_{113}$, $P_{114}$, $P_{121}$, $P_{131}$, $P_{141}$, $P_{211}$, $P_{311}$, $P_{313}$, $P_{333}$, $P_{411}$, $P_{414}$, $P_{441}$, $P_{444}$) and for each orientation and the received electric field strength,

the position determination unit (594) is configured to determine a position and orientation of the capsule endoscope (3) in the first estimation step, based on a comparison result by the electric field strength comparing unit (593), wherein the determined position is the center position of the subregion ($P_{111}$, $P_{113}$, $P_{114}$, $P_{121}$, $P_{131}$, $P_{141}$, $P_{211}$, $P_{311}$, $P_{313}$, $P_{333}$, $P_{411}$, $P_{414}$, $P_{441}$, $P_{444}$) and the determined position and orientation have the smallest one of the residual sum of squares calculated by the electric field strength comparing unit (593),

the electric field strength comparing unit (593) is configured to calculate, as a second estimation step, a residual sum of squares between each of the theoretical electric field strengths stored in the storage unit (56) for each region of a plurality of regions ($P_{311(111)}$, $P_{311(131)}$, $P_{311(331)}$, $P_{311(333)}$, $P_{311(311)}$, $P_{311(313)}$, $P_{311(113)}$) into which the subregion ($P_{311}$) including the position of the capsule endoscope (3) determined in the first estimation step is further divided and for each orientation of the capsule endoscope (3) and the received electric field strength, and

the position determination unit (594) is configured to determine as a final position and orientation of the capsule endoscope (3) in the second estimation step, the position and orientation which has the smallest sum of residual squares calculated by the electric field strength comparing unit (593).

2. The position detecting apparatus of the capsule endoscope (1) according to claim 1, further comprising a trajectory calculation unit (595) configured to calculate a trajectory of the capsule endoscope (3), wherein

the position determination unit (594) is configured to extract, for each image data, a specified number of regions and orientations in ascending order of the residual sums of squares, as candidate positions of the position and orientation of the capsule endoscope (3),

the position determination unit (594) is configured to store the candidate positions into the storage unit (56),

the trajectory calculation unit (595) is configured to calculate a distance (d) between the candidate positions of the image data taken at a previous timing ($t_n$) and a subsequent timing ($t_{n+1}$), to compare the calculated distance to a movable distance that the capsule endoscope (3) can move inside the subject (2) within a time interval for the position estimation of the capsule endoscope (3), the time interval being pre-stored in the storage unit (56), to store the candidate positions (G) where the distance is smaller than the movable distance and is the smallest into the storage unit (56), and to calculate the trajectory of the capsule endoscope (3) using the candidate positions (G) where the distance is smaller than the movable distance and is the smallest, and

the position determination unit (594) is configured to determine the position and orientation of the capsule endoscope (3) where each image data has been taken, based on the trajectory of the capsule endoscope (3).

3. The position detecting apparatus of the capsule endoscope (1) according to claim 1, wherein the receiving antenna unit (4) has a sheet shape on which the plurality of receiving antennas (40a-40h) are provided.

4. A capsule endoscope system (1) comprising:

a capsule endoscope (3) configured to obtain image data of an inside of a subject (2);

the position detecting apparatus according to claim 1 configured to receive the image data transmitted from the capsule endoscope (3) and to estimate a position and orientation of the capsule endoscope (3) where the received image data has been taken; and

an image display unit (6) configured to obtain the image data and position information of the image data from the receiving antenna (4) and the position detecting apparatus and to display the obtained image data and position information.

5. The capsule endoscope system (1) according to claim 4, wherein

the position detecting apparatus includes a trajectory calculation unit (595) configured to calculate a trajectory of the capsule endoscope (3) based on the position of the capsule endoscope (3) determined by the position determination unit (594), and

the image display unit (6) is configured to display the image data and the trajectory in the subject (2) of the capsule endoscope (3) calculated by the trajectory calculation unit (595).

**Patentansprüche**

1. Positionserkennungsvorrichtung eines Kapselendoskops (1) umfassend:

eine Empfangsantenneneinheit (4), die eingerichtet ist, mittels einer Mehrzahl von Empfangsantennen (40a-40h) ein von einem Kapselendoskops (3) innerhalb eines Subjekts (2) übertragenes drahtloses Signal, zu empfangen;

eine Speichereinheit (56), die eingerichtet ist, eine theoretische elektrische Feldstärke des drahtlosen Signals, die von jeder der Empfangsantennen (40a-40h) empfangen wird, zu speichern, wobei die theoretische elektrische Feldstärke von einer Position und Orientierung des Kapselendoskops (3) innerhalb des Subjekts (2) abhängt;

eine Elektrischefeldstärkenvergleichseinheit (593), die eingerichtet ist, einen vorgegebenen Wert zu vergleichen, der berechnet wird, indem eine Differenz zwischen einer empfangenen elektrischen Feldstärke des drahtlosen Signals, die von jeder der Empfangsantennen (40a-40h) empfangen wird, und der in der Speichereinheit (56) gespeicherten theoretischen elektrischen Feldstärke verwendet wird;

eine Positionsbestimmungseinheit (594), die eingerichtet ist, basierend auf einem Vergleichsergebnis der Elektrischefeldstärkenvergleichseinheit (593) eine Position und eine Orientierung des Kapselendoskops (3) zu bestimmen, wo Bilddaten aufgenommen worden sind, **dadurch gekennzeichnet, dass**

die Speichereinheit (56) eine theoretische elektrischen Feldstärke entsprechend einer Orientierung des Kapselendoskops (3) für jede einer Mehrzahl von Unterregionen ($P_{111}$, $P_{113}$, $P_{114}$, $P_{121}$, $P_{131}$, $P_{141}$, $P_{211}$, $P_{311}$, $P_{313}$, $P_{333}$, $P_{411}$, $P_{414}$, $P_{441}$, $P_{444}$), in welche eine Auftrittsregion (T) innerhalb des Subjekts (2), wo das Kapselendoskop (3) vorhanden sein kann, unterteilt ist, und für jede Region, in welche jede Unterregion weiter unterteilt ist, speichert;

die Elektrischefeldstärkenvergleichseinheit (593) eingerichtet ist, als einen ersten Schätzschritt eine Residuenquadratsumme zwischen jeder der theoretischen elektrischen Feldstärken, die in der Speichereinheit (56) für jede Mittenposition der Unterregionen ($P_{111}$, $P_{113}$, $P_{114}$, $P_{121}$, $P_{131}$, $P_{141}$, $P_{211}$, $P_{311}$, $P_{313}$, $P_{333}$, $P_{411}$, $P_{414}$, $P_{441}$, $P_{444}$) und für jede Orientierung gespeichert sind, und der empfangenen elektrischen Feldstärke zu berechnen,

die Positionsbestimmungseinheit (594) eingerichtet ist, eine Position und Orientierung des Kapselendoskops (3) in dem ersten Schätzschritt basierend auf einem Vergleichsergebnis der Elektrischefeldstärkenvergleichseinheit (593) zu bestimmen, wobei die bestimmte Position die Mittenposition der Unterregion ($P_{111}$, $P_{113}$, $P_{114}$, $P_{121}$, $P_{131}$, $P_{141}$, $P_{211}$, $P_{311}$, $P_{313}$, $P_{333}$, $P_{411}$, $P_{414}$, $P_{441}$, $P_{444}$) ist und die bestimmte Position und Orientierung die geringste von der Elektrischefeldstärkenvergleichseinheit (593) berechnete Residuenquadratsumme haben,

die Elektrischefeldstärkenvergleichseinheit (593) eingerichtet ist, als einen zweiten Schätzschritt eine Residuenquadratsumme zwischen jeder der theoretischen elektrischen Feldstärken, die in der Speichereinheit (56) für jede Region einer Mehrzahl von Regionen ($P_{311(111)}$, $P_{311(131)}$, $P_{311(331)}$, $P_{311(333)}$, $P_{311(311)}$, $P_{311(313)}$, $P_{311(113)}$), in welche die Unterregion ($P_{311}$), die die im ersten Schätzschritt bestimmte Position des Kapselendoskops (3) beinhaltet, weiter unterteilt ist, und für jede Orientierung des Kapselendoskops (3) gespeichert ist, und der empfangenen elektrischen Feldstärke zu berechnen, und

die Positionsbestimmungseinheit (594) eingerichtet ist, als eine finale Position und Orientierung des Kapselendoskops (3) in dem zweiten Schätzschritt die Position und Orientierung zu bestimmen, welche die geringste von der Elektrischefeldstärkenvergleichseinheit (593) berechnete Summe von Residuenquadraten aufweist.

2. Positionserkennungsvorrichtung des Kapselendoskops (1) nach Anspruch 1 ferner umfassend eine Trajektorienberechnungseinheit (595), die eingerichtet ist eine Trajektorie des Kapselendoskops (3) zu berechnen, wobei

die Positionsbestimmungseinheit (594) eingerichtet ist, für jede Bilddaten eine festgelegte Anzahl von Regionen und Orientierungen in aufsteigender Reihenfolge der Residuenquadratsummen als Positionskandidaten der Position und Orientierung des Kapselendoskops (3) zu extrahieren,

die Positionsbestimmungseinheit (594) eingerichtet ist, die Positionskandidaten in der Speichereinheit (56) zu speichern,

die Trajektorienberechnungseinheit (595) eingerichtet ist, eine Distanz (d) zwischen den Positionskandidaten der zu einem vorherigen Zeitpunkt ($t_n$) und einem nachfolgenden Zeitpunkt ($t_{n+1}$) aufgenommen Bilddaten zu berechnen, die berechnete Distanz mit einer zurücklegbaren Distanz, die das Kapselendoskop (3) innerhalb des Subjekts (2) innerhalb eines Zeitintervalls für die Positionsschätzung des Kapselendoskops (3) zurücklegen kann, wobei das Zeitintervall in der Speichereinheit (56) vorgespeichert ist, die Positionskandidaten (G), wo die Distanz kleiner als die zurücklegbare Distanz ist und am kleinsten ist, in der Speichereinheit (56) zu speichern und die Trajektorie des Kapselendoskops (3) unter Verwendung des Positionskandidaten (G), wo die Distanz kleiner als die zurücklegbare Distanz ist und am kleinsten ist, zu berechnen, und

die Positionsbestimmungseinheit (594) eingerichtet ist, basierend auf der Trajektorie des Kapselendoskops (3) die Position und Orientierung des Kapselendoskops (3), wo jede Bilddaten aufgenommen worden sind, zu bestimmen.

**3.** Positionserkennungsvorrichtung des Kapselendoskops (1) nach Anspruch 1, wobei die Empfangsantenneneinheit (4), eine Plattenform aufweist, auf der die Mehrzahl von Empfangsantennen (40a-40h) angeordnet ist.

**4.** Kapselendoskopsystem (1) umfassend:

ein Kapselendoskop (3), das eingerichtet ist, Bilddaten des Inneren eines Subjekts (2) zu gewinnen;
die Positionserkennungsvorrichtung nach Anspruch 1, die eingerichtet ist, die von dem Kapselendoskop (3) übertragenen Bilddaten zu empfangen und eine Position und Orientierung des Kapselendoskops (3), wo die empfangenen Bilddaten aufgenommen worden sind, zu schätzen; und
eine Bildanzeigeeinheit (6), die eingerichtet ist, die Bilddaten und die Positionsinformation der Bilddaten von der Empfangsantenne (4) und der Positionserkennungsvorrichtung zu erhalten und die erhaltenen Bilddaten und die erhaltene Positionsinformation anzuzeigen.

**5.** Kapselendoskopsystem (1) nach Anspruch 4, bei dem
die Positionserkennungsvorrichtung eine Trajektorienberechnungseinheit (595) umfasst, die eingerichtet ist, eine Trajektorie des Kapselendoskops (3) basierend auf der von der Positionsbestimmungseinheit (594) bestimmten Position des Kapselendoskops (3) zu berechnen, und
die Bildanzeigeeinheit (6) eingerichtet ist, die Bilddaten und die von der Trajektorienberechnungseinheit (595) berechnete Trajektorie innerhalb des Subjeckts (2) des Kapselendoskops (3) anzuzeigen.

## Revendications

**1.** Appareil de détection de position d'un endoscope (1) de type capsule comprenant :

une unité (4) d'antennes de réception configurée pour recevoir, par une pluralité d'antennes de réception (40a à 40h), un signal sans fil transmis depuis un endoscope (3) de type capsule à l'intérieur d'un sujet (2) ;
une unité (56) de mémoire configurée pour mémoriser une intensité de champ électrique théorique du signal sans fil reçu par chacune des antennes de réception (40a à 40h), l'intensité de champ électrique théorique dépendant d'une position et d'une orientation de l'endoscope (3) de type capsule dans le sujet (2) ;
une unité (593) de comparaison d'intensité de champ électrique configurée pour comparer une valeur spécifiée calculée en utilisant une différence entre une intensité de champ électrique reçue du signal sans fil reçu par chacune des antennes de réception (40a à 40h) et l'intensité de champ électrique théorique mémorisée dans l'unité (56) de mémoire ; et
une unité (594) de détermination de position configurée pour déterminer une position et une orientation de l'endoscope (3) de type capsule où les données d'image ont été prises, sur la base d'un résultat de comparaison par l'unité (593) de comparaison d'intensité de champ électrique, **caractérisé en ce que**
l'unité (56) de mémoire mémorise une intensité de champ électrique théorique conformément à une orientation de l'endoscope (3) de type capsule pour chacune parmi une pluralité de sous-régions ($P_{111}$, $P_{113}$, $P_{114}$, $P_{121}$, $P_{131}$, $P_{141}$, $P_{211}$, $P_{311}$, $P_{313}$, $P_{333}$, $P_{411}$, $P_{414}$, $P_{441}$, $P_{444}$) dans lesquelles une région d'occurrence (T) à l'intérieur du sujet (2) où l'endoscope (3) peut être présent est divisée, et pour chaque région dans laquelle chaque sous-région est en outre divisée,
l'unité (593) de comparaison d'intensité de champ électrique est configurée pour calculer, en tant que première étape d'estimation, une somme résiduelle des carrés entre chacune des intensités de champ électrique théorique mémorisées dans l'unité (56) de mémoire pour chaque position centrale des sous-régions ($P_{111}$, $P_{113}$, $P_{114}$, $P_{121}$, $P_{131}$, $P_{141}$, $P_{211}$, $P_{311}$, $P_{313}$, $P_{333}$, $P_{411}$, $P_{414}$, $P_{441}$, $P_{444}$) et pour chaque orientation, et l'intensité de champ électrique reçue,
l'unité (594) de détermination de position est configurée pour déterminer une position et une orientation de l'endoscope (3) de type capsule à la première étape d'estimation, sur la base d'un résultat de comparaison par l'unité (593) de comparaison d'intensité de champ électrique, où la position déterminée correspond à la position centrale de la sous-région ($P_{111}$, $P_{113}$, $P_{114}$, $P_{121}$, $P_{131}$, $P_{141}$, $P_{211}$, $P_{311}$, $P_{313}$, $P_{333}$, $P_{411}$, $P_{414}$, $P_{441}$, $P_{444}$) et la position et l'orientation déterminées présentent la plus petite des sommes résiduelles des carrés calculées par l'unité (593) de comparaison d'intensité de champ électrique,
l'unité (593) de comparaison d'intensité de champ électrique est configurée pour calculer, en tant que deuxième étape d'estimation, une somme résiduelle des carrés entre chacune des intensités de champ électrique théoriques mémorisées dans l'unité (56) de mémoire pour chaque région parmi une pluralité de régions ($P_{311(111)}$, $P_{311(131)}$, $P_{311(331)}$, $P_{311(333)}$, $P_{311(311)}$, $P_{311(313)}$, $P_{311(113)}$) dans lesquelles la sous-région ($P_{311}$) incluant la position de l'endoscope (3) de type capsule déterminée à la première étape d'estimation est en outre divisée et pour chaque orientation de l'endoscope (3) de type capsule et l'intensité de champ électrique reçue, et l'unité (594) de détermination de position est configurée pour déterminer en tant que position et orientation finales de l'endoscope (3) de type capsule à la deuxième étape d'estimation, la position et l'orientation présentant la plus petite somme résiduelle des carrés calculée par l'unité (593) de comparaison d'intensité de champ électrique.

2. Appareil de détection de position de l'endoscope (1) de type capsule selon la revendication 1, comprenant en outre une unité (595) de calcul de trajectoire configurée pour calculer une trajectoire de l'endoscope (3) de type capsule, dans lequel
l'unité (594) de détermination de position est configurée pour extraire, pour chaque donnée d'image, un nombre spécifié de régions et d'orientations en ordre croissant des sommes résiduelles des carrés, en tant que positions candidates de la position et de l'orientation de l'endoscope (3) de type capsule,
l'unité (594) de détermination de position est configurée pour mémoriser les positions candidates dans l'unité (56) de mémoire,
l'unité (595) de calcul de trajectoire est configurée pour calculer une distance (d) entre les positions candidates des données d'images prises à un moment précédant ($t_n$) et un moment suivant ($t_{n+1}$), pour comparer la distance calculée à une distance de déplacement de laquelle l'endoscope (3) de type capsule peut se déplacer à l'intérieur du sujet (2) pendant un intervalle de temps pour l'estimation de position de l'endoscope (3) de type capsule, l'intervalle de temps étant pré-mémorisé dans l'unité (56) de mémoire, pour mémoriser les positions candidates (G) où la distance est plus petite que la distance de déplacement et est la plus petite dans l'unité (56) de mémoire, et pour calculer la trajectoire de l'endoscope (3) de type capsule en utilisant les positions candidates (G) où la distance est plus petite que la distance de déplacement et est la plus petite, et
l'unité (594) de détermination de position est configurée pour déterminer la position et l'orientation de l'endoscope (3) de type capsule où chaque donnée d'image a été prise, sur la base de la trajectoire de l'endoscope (3) de type capsule.

3. Appareil de détection de position de l'endoscope (1) de type capsule selon la revendication 1, dans lequel l'unité (4) d'antennes de réception présente une forme de feuille sur laquelle la pluralité d'antennes de réception (40a à 40h) sont prévues.

4. Système (1) d'endoscope de type capsule comprenant :

un endoscope (3) de type capsule configuré pour obtenir des données d'image de l'intérieur d'un sujet (2) ;
l'appareil de détection de position selon la revendication 1 configuré pour recevoir les données d'image transmises depuis l'endoscope (3) de type capsule et pour estimer une position et une orientation de l'endoscope (3) de type capsule où les données d'image reçues ont été prises ; et
une unité (6) d'affichage d'image configurée pour obtenir les données d'image et des informations de position des données d'image depuis l'antenne de réception (4) et l'appareil de détection de position et pour afficher les informations de position et les données d'image obtenues.

5. Système (1) d'endoscope de type capsule selon la revendication 4, dans lequel
l'appareil de détection de position comprend une unité (595) de calcul de trajectoire configurée pour calculer une trajectoire de l'endoscope (3) de type capsule sur la base de la position de l'endoscope (3) de type capsule déterminée par l'unité (594) de détermination de position, et
l'unité (6) d'affichage d'image est configurée pour afficher les données d'image et la trajectoire dans le sujet (2) de l'endoscope (3) de type capsule calculée par l'unité (595) de calcul de trajectoire.

# FIG.1

# FIG.2

**FIG.3**

# FIG.4A

# FIG.4B

# FIG.5

# FIG.6

# FIG.7

# FIG.8A

# FIG.8B

FIG.9

# FIG.10

- START
- EXTRACT CANDIDATE POSITION — S11
- CALCULATE CONNECTION INFORMATION — S12
- TRAJECTORY ESTIMATION PROCESS — S13
- END

# FIG.11

$d((n-1)1, n1)$

$d(n1, (n+1)1)$

$G_{n1}$

$G_{(n+1)1}$

$G_{n2}$

$d((n-1)2, n1)$

$G_{(n-1)1}$

$G_{(n+1)2}$

$G_{(n+1)3}$

$G_{n3}$

$d((n-1)3, n1)$

$G_{(n-1)2}$

$G_{(n-1)3}$

TIME $t_n$

TIME $t_{n+1}$

TIME $t_{n-1}$

# FIG.12

START

m=n-1(t=t$_{n-1}$) — S21

i=1 — S22

READ OUT CANDIDATE POSITION Gmi AND CONNECTION INFORMATION FROM STORAGE UNIT — S23

IS CONNECTION INFORMATION PRESENT? — S24

NO

YES

IS CONNECTED WITH CANDIDATE POSITION G(m+1)j? — S25

NO

YES

STORE AS NEW PATH INFORMATION IN STORAGE UNIT — S27

STORE AS PATH INFORMATION IN STORAGE UNIT — S26

i<3? — S28

YES

NO

i=i+1 — S29

2<m? — S30

YES

NO

m=m-1 — S31

END

# FIG.13A

# FIG.13B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007000608 A
- JP 2010524557 A
- EP 2008572 A1
- EP 1884184 A1
- EP 2082680 A1
- US 20080242931 A1